# FASCICULE DE BREVET EUROPEEN

(11) **EP 0 697 210 B1**
(45) Date de publication et mention de la délivrance du brevet: **25.09.1996**
(21) Numéro de dépôt: 95401500.4
(22) Date de dépôt: 23.06.1995
(51) Int. Cl.: A61K 7/13

(54) **Utilisation d'un sel de manganèse, de zinc et/ou de cobalt dans un procédé de teinture en un temps mettant en oeuvre un composé indolique et/ou indolinique**
Verwendung eines Mangan, Zinc und/oder Kobaltsalzes in einem zweistufigen Färbeverfahren mit einer Indol-/Indolinverbindung
Use of a manganese, zinc and/or cobalt salt in a two-steps dyeing process based on an ondole or indoline compound

(30) Priorité: 22.07.1994 FR 9409117
(43) Date de publication de la demande: 21.02.1996
(73) Titulaire: L'OREAL, F-75008 Paris (FR)
(72) Inventeur: Samain, Henri, F-91570 Bievres (FR)
(74) Mandataire: Andral, Christophe André Louis

(56) Documents cités:
- EP-A- 0 621 029
- WO-A-93/18738
- FR-A- 1 133 594

## Description

La présente invention a pour objet l'utilisation d'un sel de manganèse, de zinc et / ou de cobalt dans un procédé de teinture des fibres kératiniques en un temps mettant en oeuvre un composé indolique et/ou indolinique.

La coloration des cheveux, de la peau et des poils, en particulier d'origine humaine, provient principalement des pigments mélaniques sécrétés par les mélanocytes. Ces pigments d'origine naturelle, comprennent en particulier des eumélanines. Leur biosynthèse naturelle s'effectue en plusieurs étapes par polymérisation des produits d'oxydation d'un acide aminé : la tyrosine. L'un des produits d'oxydation de la tyrosine est le 5,6-dihydroxyindole qui polymérise à son tour en eumélanines.

On a déjà proposé dans le passé de repigmenter les cheveux humains qui ont tendance à blanchir ou à grisonner avec du 5,6-dihydroxyindole. Le 5,6-dihydroxyindole est généralement utilisé dans des compositions aqueuses avec lesquelles il est possible de teindre les fibres kératiniques et en particulier les cheveux, cette teinture pouvant être effectuée en plusieurs fois c'est à dire de façon progressive sans autre agent oxydant que l'oxygène de l'air. Il est possible de cette façon de repigmenter les cheveux en partant de nuances relativement claires par une application du produit, jusque dans des nuances de plus en plus soutenues, par superposition des applications.

Il est également connu selon la demande FR-A-1 133 594 d'ajouter des catalyseurs d'oxydation, notamment des sels de manganèse ou de cobalt, dans une composition tinctoriale contenant du 5,6-dihydroxyindole, afin d'obtenir des nuances plus fortes.

On recherche, lors de la coloration des fibres kératiniques et plus particulièrement des cheveux, avec le 5,6-dihydroxyindole ou la 5,6-dihydroxyindoline ou leurs dérivés, l'obtention de nuances plus ou moins sombres et neutres qui n'évoluent pas au cours du temps, notamment lors de l'exposition à la lumière, à la transpiration ou à des lavages répétés.

En particulier, il faut noter que les nuances de gris peu soutenu ont tendance à évoluer au cours du temps de façon plus rapide et plus marquée que les nuances sombres.

Dans la demande de brevet FR-A-2 700 266, il a été proposé l'utilisation d'un sel de magnésium dans un procédé de teinture des cheveux mettant en oeuvre un composé indolique pour améliorer la ténacité des colorations vis à vis de la lumière. Cependant l'utilisation des sels de magnésium n'est pas entièrement satisfaisante notamment en ce qui concerne les nuances gris clair qui ont malgré cela tendance à évoluer au cours du temps.

C'est afin de résoudre ce problème que la demanderesse a découvert ce qui fait l'objet de l'invention.

L'invention a donc pour objet l'utilisation d'un sel de manganèse, de zinc et / ou de cobalt, dans un procédé de teinture en un temps des fibres kératiniques et en particulier des fibres kératiniques humaines telles que les cheveux mettant en oeuvre un composé indolique et/ou indolinique, afin d'obtenir des colorations dans des nuances grises n'évoluant pas au cours du temps.

La demanderesse a remarqué en particulier que les colorations obtenues sont particulièrement résistantes à la lumière et ceci de façon supérieure à l'état de la technique, c'est à dire que la lumière n'entraîne pas une dégradation importante des colorations obtenues. Ces colorations présentent également de bonnes résistances aux lavages répétés et à la transpiration.

L'utilisation conforme à la présente invention est plus particulièrement adaptée à l'obtention de nuances gris clair.

Selon l'invention, les sels de manganèse, de zinc et / ou de cobalt sont présents, sous forme solubilisée ou dispersée, dans la composition contenant le composé indolique et/ou indolinique.

Les sels de manganèse, de zinc et / ou de cobalt peuvent être utilisés seuls ou en mélanges et sont plus particulièrement choisis parmi les acétates, les halogénures, les sulfates et les carbonates de manganèse, de zinc et / ou de cobalt ainsi que leurs hydrates.

Parmi ces composés, on peut citer à titre d'exemple, le diacétate de manganèse tétrahydrate, le dichlorure de manganèse et ses hydrates, le dihydrogénocarbonate de manganèse, l'acétylacétonate de manganèse, le triacétate de manganèse et ses hydrates, le trichlorure de manganèse, le dichlorure de zinc, le diacétate de zinc dihydrate, le carbonate de zinc, le dinitrate de zinc, le sulfate de zinc, le diacétate de cobalt tétrahydrate, le carbonate de cobalt, le dichlorure de cobalt, le dinitrate de cobalt, le sulfate de cobalt heptahydrate.

Le diacétate de manganèse tétrahydrate et le dichlorure de zinc sont particulièrement préférés.

Les sels de manganèse, de zinc et / ou de cobalt utilisés selon l'invention sont généralement mis en oeuvre dans des proportions comprises entre 0,001 et 4 % en poids d'équivalent métal par rapport au poids total de la composition et de préférence entre 0,005 et 2 % en poids d'équivalent métal par rapport au poids total de la composition.

Selon l'invention, les composés indoliques utilisables répondent à la formule (I) suivante : 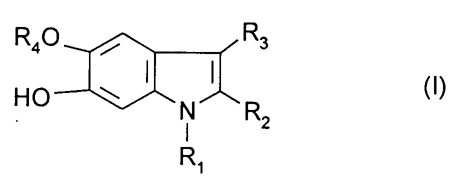 dans laquelle :
R₁, R₂, R₃, R₄; identiques ou différents, désignent un atome d'hydrogène ou un radical alkyle en C₁-C₄, R₁, R₂, R₃ désignant hydrogène lorsque R₄ désigne un radical alkyle en C₁-C₄, ainsi que les sels d'addition de ces composés avec un acide.

Dans la formule (I) ci-dessus, le radical alkyle désigne de préférence méthyle ou éthyle et R₁, R₂ et R₃ ne désignent pas simultanément un radical alkyle en C₁-C₄.

Les composés de formule (I) préférés sont choisis parmi le 5,6-dihydroxyindole, le 2-méthyl 5,6-dihydroxyindole, le 3-méthyl 5,6-dihydroxyindole, le 1-méthyl 5,6-dihydroxyindole, le 2,3-diméthyl 5,6-dihydroxyindole, le 5-méthoxy 6-hydroxyindole, et leurs sels d'addition avec un acide. Un composé particulièrement préféré est le 5,6-dihydroxyindole.

Selon l'invention, les composés indoliniques utilisables répondent à la formule (II) suivante : 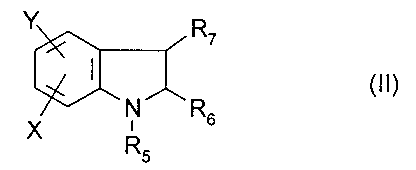 dans laquelle :
- R₅ et R₇, identiques ou différents, représentent un atome d'hydrogène ou un radical alkyle en C₁-C₄ ;
- R₆ représente un atome d'hydrogène, un radical alkyle en C₁-C₄ ou -COOH ;
- X désigne un atome d'hydrogène, un radical amino, hydroxyle, alkyle en C₁-C₄ ou alcoxy en C₁-C₄ ;
- Y désigne un radical hydroxyle ou amino ;
sous réserve que lorsque X désigne un radical hydroxyle ou un radical alkyle, X occupe les positions 5, 6 ou 7 et est en position ortho par rapport à Y, ainsi que les sels d'addition de ces composés avec un acide.

Parmi les composés indoliniques de formule (II) préférés, on peut citer la 5,6-dihydroxyindoline, la N-méthyl 5,6-dihydroxyindoline, la N-éthyl 5,6-dihydroxyindoline, la N-butyl 5,6-dihydroxyindoline, la 4-hydroxy 5-méthoxyindoline, la 6-hydroxy 7-méthoxyindoline, la 6,7-dihydroxyindoline, la 4,5-dihydroxyindoline, la 5-méthoxy 6-hydroxyindoline, et leurs sels d'addition avec un acide.

Les composés indoliques et/ou indoliniques utilisés selon l'invention, sont généralement mis en oeuvre dans des proportions suffisantes pour repigmenter les fibres kératiniques et en particulier les cheveux. Ces proportions sont de préférence comprises entre 0,1 et 5 % en poids et encore plus préférentiellement entre 0,2 et 3 % en poids par rapport au poids total de la composition.

Les compositions utilisées selon l'invention sont des compositions aqueuses ou des compositions dont le milieu est un mélange d'eau et d'un solvant cosmétiquement acceptable.

Lorsque les compositions contiennent un solvant, celui-ci peut être choisi parmi les alcools inférieurs en C₁-C₄ tels que l'éthanol, l'alcool propylique, l'alcool isopropylique, l'alcool tertiobutylique ; les glycols tels que l'éthylèneglycol, le propylèneglycol ; les éthers de glycols tels que les éthers monométhylique, monoéthylique ou monobutylique de l'éthylèneglycol, les monométhyléthers du propylèneglycol et du dipropylèneglycol ; les esters inférieurs comme le lactate de méthyle et l'acétate de monoéthyléther de l'éthylèneglycol.

Les solvants particulièrement préférés sont choisis parmi l'éthanol et le propylèneglycol.

Lorsqu'ils sont présents, les solvants représentent de préférence de 0,5 à 50 % en poids et de préférence de 2 à 20 % en poids par rapport au poids total de la composition.

Les compositions utilisées selon l'invention, ont généralement un pH compris entre 5 et 10 et de préférence entre 7 et 9,5. Ce pH est ajusté à l'aide d'agents alcalinisants ou acidifiants classiquement utilisés dans le domaine de la teinture des cheveux.

Parmi les agents alcalinisants, on peut citer à titre d'exemple, l'ammoniaque, les alcanolamines telles que par exemple les mono-, di- et triéthanolamines et leurs dérivés, les hydroxydes de sodium ou de potassium tels que par exemple la soude et la potasse, les alkylamines, les carbonates alcalins ou d'ammonium.

Parmi les agents acidifiants, on peut citer à titre d'exemple, l'acide orthophosphorique, l'acide lactique, l'acide acétique, l'acide tartrique, l'acide chlorhydrique et l'acide citrique.

Les compositions mises en oeuvre selon l'invention peuvent se présenter sous la forme de lotions plus ou moins épaissies, de gels, de shampooings, d'émulsions telles que des laits et des crèmes ou elles peuvent être propulsées sous forme de mousses aérosols.

Les compositions contenant le sel de manganèse, de zinc et / ou de cobalt ainsi que le composé indolique et/ou indolinique peuvent contenir en outre au moins un adjuvant choisi parmi les adjuvants utilisés classiquement dans les compositions pour la teinture des cheveux tels que les agents tensio-actifs anioniques, cationiques, nonioniques, amphotères, zwittérioniques ou leurs mélanges, les agents épaississants minéraux ou organiques, les agents antioxydants, les agents de pénétration, les agents séquestrants, les parfums, les tampons, les agents dispersants, les agents de conditionnement, les agents filmogènes, les agents conservateurs, les agents opacifiants, les filtres solaires, etc...

Selon l'invention, les compositions contenant le sel de manganèse, de zinc et / ou de cobalt ainsi que le composé indolique et/ou indolinique sont appliquées sur les fibres kératiniques et en particulier sur les cheveux avec un temps de pause généralement compris entre 15 secondes et 30 minutes. Les cheveux sont ensuite rincés à l'eau puis séchés. Si on le désire, un shampooing peut suivre l'application de la composition. Le pH de ce shampooing est de préférence compris entre 5 et 11.

Selon l'invention, le procédé de teinture utilisant un sel de manganèse, de zinc et / ou de cobalt est mis en oeuvre en n'utilisant pas d'autre oxydant que l'oxygène de l'air.

Les exemples suivants sont destinés à illustrer l'invention sans pour autant en limiter la portée.

### EXEMPLES

### EXEMPLE 1 COMPARATIF

On a préparé la composition A suivante, ne faisant pas partie de l'invention :

| | |
|---|---|
| - 5,6-dihydroxyindole | 0,5 g |
| - Gomme de xanthane | 1,8 g |
| - Alkylpolyglucoside en solution aqueuse à 60 % de matière active (M.A.) vendu par la société SEPPIC | 4 g |
| - Acide tartrique | 0,35 g |
| - Ethanol | 12 g |
| - Triéthanolamine q.s.p. | pH 8,5 |
| - Eau déminéralisée q.s.p. | 100 g |

On a préparé la composition B suivante, faisant partie de l'invention :

| | |
|---|---|
| - 5,6-dihydroxyindole | 0,5 g |
| - **Diacétate de manganèse, tétrahydrate (soit 0,2 % en poids d'équivalent métal)** | **1 g** |
| - Gomme de xanthane | 1,8 g |
| - Alkylpolyglucoside en solution aqueuse à 60 % de matière active (M.A.) vendu par la société SEPPIC | 4 g |
| - Acide tartrique | 0,35 g |
| - Ethanol | 12 g |
| - Triéthanolamine q.s.p. | pH 8,5 |
| - Eau déminéralisée q.s.p. | 100 g |

On a appliqué les compositions A et B sur des mèches de cheveux gris naturels à 90 % de blancs pendant 10 minutes puis on a rincé les cheveux. On a effectué ensuite pendant environ 30 secondes un shampooing de composition suivante :

| | |
|---|---|
| - Monoisopropanolamide d'acide de coprah vendu par la société ALLBRIGHT ET WILSON SAINT MIHIEL | 2,5 g |
| - Lauryléthersulfate de monoéthanolamine en solution aqueuse à 28 % de M.A. | 40 g |
| - Cocoylbétaïne en solution aqueuse à 32 % de M.A. vendue par la société HENKEL | 10 g |
| - Monosulfosuccinate de monoéthanolamide ricinoléïque, sel disodique vendu par la société WITCO | 2 g |
| - Hydroxyde de sodium q.s.p. | pH 9,5 |
| - Parfum q.s. | |
| - Eau déminéralisée q.s.p. | 100 g |

Les cheveux ont ensuite été rincés et séchés.

Les deux mèches de cheveux étaient teintes dans une nuance proche gris métallique.

Les deux mèches de cheveux ont ensuite été laissées à l'air libre et à la lumière naturelle pendant une période de trois semaines.
On a alors observé que la mèche de cheveux teinte par la composition A ne faisant pas partie de l'invention était devenue gris doré alors que la mèche de cheveux teinte par la composition B faisant partie de l'invention présentait toujours une couleur gris métallique n'ayant pas évolué.

### EXEMPLE 2 COMPARATIF

On a préparé la composition C suivante, faisant partie de l'invention :

| | |
|---|---|
| - 5,6-dihydroxyindole | 0,5 g |
| - **Diacétate de manganèse, tétrahydrate (soit 0,02 % en poids d'équivalent métal)** | **0,1 g** |
| - Gomme de xanthane | 1,8 g |
| - Alkylpolyglucoside en solution aqueuse à 60 % de matière active (M.A.) vendu par la société SEPPIC | 4 g |
| - Acide tartrique | 0,35 g |
| - Ethanol | 12 g |
| - Triéthanolamine q.s.p. | pH 8,5 |
| - Eau déminéralisée q.s.p. | 100 g |

Sur une tête portant environ 70 % de cheveux blancs, on a appliqué, sur la première demi-tête, environ 15 g de la composition A telle qu'utilisée à l'exemple 1 ci-dessus et ne faisant pas partie de l'invention et sur la seconde demi-tête, environ 15 g de la composition C ci-dessus faisant partie de l'invention.
On a laissé poser ces compositions pendant environ 10 minutes puis on a rincé les cheveux.
On a ensuite effectué un shampooing de composition identique à celui de l'exemple 1 ci-dessus que l'on a émulsionné pendant environ 30 secondes.
Les cheveux ont ensuite été rincés et séchés.
Les cheveux initialement blancs sont devenus légèrement gris, de façon identique sur les deux demi-têtes.

Une semaine après cette première application des compositions A et C, une deuxième application a été réalisée de façon identique.
Les cheveux sont devenus nettement gris métallique, de façon identique sur chaque demi-tête.

Au bout de trois semaines, et après que le modèle se soit lavé les cheveux neuf fois sans qu'aucune application des compositions A ou C ne soit intervenue, on a observé que les cheveux de la demi-tête ayant subi l'application de la composition A ne faisant pas partie de l'invention étaient devenus gris doré alors que les cheveux de la demi-tête ayant subi l'application de la composition C faisant partie de l'invention présentaient toujours une couleur gris métallique n'ayant pas évolué.

### EXEMPLE 3 COMPARATIF

On a préparé la composition D suivante, faisant partie de l'invention:

| | |
|---|---|
| - 5,6-dihydroxyindole | 0,5 g |
| - **Dichlorure de zinc** **(*)** | **1 g** |
| - Gomme de xanthane | 1,8 g |
| - Alkylpolyglucoside en solution aqueuse à 60 % de matière active (M.A.) vendu par la société SEPPIC | 4 g |
| - Acide tartrique | 0,35 g |
| - Ethanol | 12 g |
| - Triéthanolamine q.s.p. | pH 8,5 |
| - Eau déminéralisée q.s.p. | 100 g |

| | |
|---|---|
| (*) On a introduit le dichlorure de zinc au moment de l'emploi dans la composition D ci-dessus. | |

La composition A de l'exemple 1 et la composition D ci-dessus ont été appliquées pendant environ 10 minutes sur des mèches de cheveux gris naturels à 90 % de blancs. Les cheveux ont ensuite été rincés et lavés avec le shampooing de l'exemple 1 qui a été émulsionné pendant environ 30 secondes. Les cheveux ont ensuite été rincés puis séchés.

On a renouvelé cette application une fois.

Les mèches ont ensuite été laissées à l'air libre et à la lumière naturelle pendant trois semaines.
L'évaluation des colorations obtenues juste après la deuxième application (t = 0) et leur évolution au bout de trois semaines (t = 3 semaines) a été mesurée dans le système Lab et figure dans le tableau ci-après:

| **Composition** | **Coloration à t = 0** | | | **Coloration à t = 3 semaines** | | | **Ecart** | | |
|---|---|---|---|---|---|---|---|---|---|
| | **L** | **a** | **b** | **L** | **a** | **b** | **L** | **a** | **b** |
| **A** | 34 | 1,8 | 3,4 | 32 | 2,3 | 5,2 | -2 | 0,5 | 1,7 |
| **C** | 29 | 1,6 | 1,2 | 29,4 | 1,3 | 2 | -0,6 | -0,3 | 0,8 |

Selon le système Lab, L indique la clarté. Plus la valeur de L est élevée, plus la couleur est claire. Inversement, plus la valeur de L est faible, plus la couleur est foncée et donc puissante.

La teinte et la saturation sont exprimées par a et b.
a et b indiquent deux axes de couleur, a l'axe rouge vert et b l'axe jaune bleu.

Une valeur positive de a correspond à une teinte rouge d'autant plus saturée que la valeur absolue de a est élevée.

Une valeur négative de a correspond à une teinte verte d'autant plus saturée que la valeur absolue de a est élevée.

Une valeur positive de b correspond à une teinte jaune d'autant plus saturée que la valeur absolue de b est élevée.

Une valeur négative de b correspond à une teinte bleue d'autant plus saturée que la valeur absolue de b est élevée.

Des valeurs proches de zéro pour a ou b correspondent à des teintes grises.

Au bout de trois semaines, les mèches teintes avec la composition D faisant partie de l'invention présentaient une couleur ayant moins évolué que celle des mèches teintes avec la composition A ne faisant pas partie de l'invention.

## Revendications

1. Utilisation d'un sel de manganèse, de zinc et / ou de cobalt dans un procédé de teinture en un temps des fibres kératiniques et en particulier des fibres kératiniques humaines telles que les cheveux mettant en oeuvre un composé indolique et/ou indolinique, afin d'obtenir des colorations dans des nuances grises n'évoluant pas au cours du temps.

2. Utilisation selon la revendication 1, caractérisée par le fait que l'on obtient des colorations dans des nuances de gris clair n'évoluant pas au cours du temps.

3. Utilisation selon la revendication 1 ou 2, caractérisée par le fait que les sels de manganèse, de zinc et / ou de cobalt sont présents, sous forme solubilisée ou dispersée, dans la composition contenant le composé indolique et/ou indolinique.

4. Utilisation selon l'une quelconque des revendications 1 à 3, caractérisée par le fait que les sels de manganèse, de zinc et / ou de cobalt sont utilisés seuls ou en mélanges et sont choisis parmi les acétates, les halogénures, les sulfates et les carbonates de manganèse, de zinc et / ou de cobalt ainsi que leurs hydrates.

5. Utilisation selon la revendication 4, caractérisée par le fait que les sels de manganèse, de zinc et / ou de cobalt sont choisis parmi le diacétate de manganèse tétrahydrate, le dichlorure de manganèse et ses hydrates, le dihydrogénocarbonate de manganèse, I'acétylacétonate de manganèse, le triacétate de manganèse et ses hydrates, le trichlorure de manganèse, le dichlorure de zinc, le diacétate de zinc dihydrate, le carbonate de zinc, le dinitrate de zinc, le sulfate de zinc, le diacétate de cobalt tétrahydrate, le carbonate de cobalt, le dichlorure de cobalt, le dinitrate de cobalt, le sulfate de cobalt heptahydrate.

6. Utilisation selon la revendication 5, caractérisée par le fait que l'on utilise le diacétate de manganèse tétrahydrate ou le dichlorure de zinc.

7. Utilisation selon l'une quelconque des revendications 1 à 6, caractérisée par le fait que les sels de manganèse, de zinc et / ou de cobalt sont mis en oeuvre dans des proportions comprises entre 0,001 et 4 % en poids d'équivalent métal par rapport au poids total de la composition.

8. Utilisation selon la revendication 7, caractérisée par le fait que les sels de manganèse, de zinc et / ou de cobalt sont mis en oeuvre dans des proportions comprises entre 0,005 et 2 % en poids d'équivalent métal par rapport au poids total de la composition.

9. Utilisation selon l'une quelconque des revendications 1 à 8, caractérisée par le fait que les composés indoliques répondent à la formule (I) suivante : 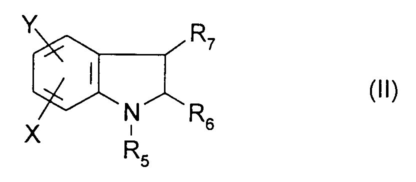 dans laquelle :
R₁, R₂, R₃, R₄; identiques ou différents, désignent un atome d'hydrogène ou un radical alkyle en C₁-C₄, R₁, R₂, R₃ désignant hydrogène lorsque R₄ désigne un radical alkyle en C₁-C₄, ainsi que les sels d'addition de ces composés avec un acide.

10. Utilisation selon la revendication 9, caractérisée par le fait que dans la formule (I) le radical alkyle désigne de préférence méthyle ou éthyle et R₁, R₂ et R₃ ne désignent pas simultanément un radical alkyle C₁-C₄.

11. Utilisation selon l'une quelconque des revendications 9 ou 10, caractérisée par le fait que les composés de formule (I) sont choisis parmi le 5,6-dihydroxyindole, le 2-méthyl 5,6-dihydroxyindole, le 3-méthyl 5,6-dihydroxyindole, le 1-méthyl 5,6-dihydroxyindole, le 2,3-diméthyl 5,6-dihydroxyindole, le 5-méthoxy 6-hydroxyindole, et leurs sels d'addition avec un acide.

12. Utilisation selon la revendication 11, caractérisée par le fait que le composé de formule (I) est le 5,6-dihydroxyindole.

13. Utilisation selon l'une quelconque des revendications 1 à 8, caractérisée par le fait que les composés indoliniques répondent à la formule (II) suivante : 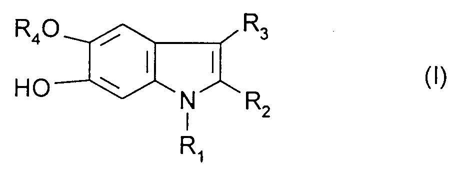 dans laquelle :
- R₅ et R₇, identiques ou différents, représentent un atome d'hydrogène ou un radical alkyle en C₁-C₄ ;
- R₆ représente un atome d'hydrogène, un radical alkyle en C₁-C₄ ou - COOH ;
- X désigne un atome d'hydrogène, un radical amino, hydroxyle, alkyle en C₁-C₄ ou alcoxy en C₁-C₄ ;
- Y désigne un radical hydroxyle ou amino ;
sous réserve que lorsque X désigne un radical hydroxyle ou un radical alkyle, X occupe les positions 5, 6 ou 7 et est en position ortho par rapport à Y, ainsi que les sels d'addition de ces composés avec un acide.

14. Utilisation selon la revendication 13, caractérisée par le fait que les composés indoliniques de formule (II) sont choisis parmi la 5,6-dihydroxyindoline, la N-méthyl 5,6-dihydroxyindoline, la N-éthyl 5,6-dihydroxyindoline, la N-butyl 5,6-dihydroxyindoline, la 4-hydroxy 5-méthoxyindoline, la 6-hydroxy 7-méthoxyindoline, la 6,7-dihydroxyindoline, la 4,5-dihydroxyindoline, la 5-méthoxy 6-hydroxyindoline, et leurs sels d'addition avec un acide.

15. Utilisation selon l'une quelconque des revendications 1 à 14, caractérisée par le fait que les composés indoliques et/ou indoliniques sont mis en oeuvre dans des proportions suffisantes pour repigmenter les fibres kératiniques.

16. Utilisation selon la revendication 15, caractérisée par le fait que les composés indoliques et/ou indoliniques sont mis en oeuvre dans des proportions comprises entre 0,1 et 5 % en poids par rapport au poids total de la composition.

17. Utilisation selon la revendication 16, caractérisée par le fait que les composés indoliques et/ou indoliniques sont mis en oeuvre dans des proportions comprises entre 0,2 et 3 % en poids par rapport au poids total de la composition.

18. Utilisation selon l'une quelconque des revendications 1 à 17, caractérisée par le fait que les compositions sont aqueuses ou constituées d'un mélange d'eau et d'un solvant cosmétiquement acceptable.

19. Utilisation selon la revendication 18, caractérisée par le fait que le solvant est choisi parmi les alcools inférieurs en C₁-C₄, les glycols, les éthers de glycols, les esters inférieurs.

20. Utilisation selon l'une quelconque des revendications 1 à 19, caractérisée par le fait que les compositions ont un pH compris entre 5 et 10.

21. Utilisation selon la revendication 20, caractérisée par le fait que les compositions ont un pH compris entre 7 et 9,5.

22. Utilisation selon l'une quelconque des revendications 1 à 21, caractérisée par le fait que les compositions se présentent sous la forme de lotions plus ou moins épaissies, de gels, de shampooings, d'émulsions ou de mousses.

23. Utilisation selon l'une quelconque des revendications 1 à 22, caractérisée par le fait que les compositions contiennent au moins un adjuvant choisi parmi les agents tensio-actifs anioniques, cationiques, nonioniques, amphotères, zwittérioniques ou leurs mélanges, les agents épaississants minéraux ou organiques, les agents antioxydants, les agents de pénétration, les agents séquestrants, les parfums, les tampons, les agents dispersants, les agents de conditionnement, les agents filmogènes, les agents conservateurs, les agents opacifiants, les filtres solaires.

24. Utilisation selon l'une quelconque des revendications 1 à 23, caractérisée par le fait que les compositions sont appliquées sur les fibres kératiniques et en particulier sur les cheveux avec un temps de pause généralement compris entre 15 secondes et 30 minutes puis les cheveux sont rincés à l'eau et séchés.

## Claims

1. Use of a manganese, zinc and/or cobalt salt in a single stage process for dyeing keratinous fibres and in particular human keratinous fibres such as hair using an indole and/or indoline compound, in order to obtain colours in grey shades which do not vary over time.

2. Use according to Claim 1, characterized in that colours are obtained in light grey shades which do not vary over time.

3. Use according to Claim 1 or 2, characterized in that the manganese, zinc and/or cobalt salts are present, in solubilized or dispersed form, in the composition containing the indole and/or indoline compound.

4. Use according to any one of Claims 1 to 3, characterized in that the manganese, zinc and/or cobalt salts are used alone or as mixtues and are chosen from manganese, zinc and/or cobalt acetates, halides, sulphates and carbonates as well as hydrates thereof.

5. Use according to Claim 4, characterized in that the manganese, zinc and/or cobalt salts are chosen from manganese diacetate tetrahydrate, manganese dichloride and hydrates thereof, manganese dihydrogen carbonate, manganese acetyl acetonate, manganese triacetate and hydrates thereof, manganese trichloride, zinc dichloride, zinc diacetate dihydrate, zinc carbonate, zinc dinitrate, zinc sulphate, cobalt diacetate tetrahydrate, cobalt carbonate, cobalt dichloride, cobalt dinitrate, cobalt sulphate heptahydrate.

6. Use according to Claim 5, characterized in that manganese diacetate tetrahydrate or zinc dichloride is used.

7. Use according to any one of Claims 1 to 6, characterized in that the manganese, zinc and/or cobalt salts are used in proportions of between 0.001 and 4 % by equivalent metal weight relative to the total weight of the composition.

8. Use according to Claim 7, characterized in that the manganese, zinc and/or cobalt salts are used in proportions of between 0.005 and 2 % by equivalent metal weight relative to the total weight of the composition.

9. Use according to any one of Claims 1 to 8, characterized in that the indole compounds correspond to the following formula (I): 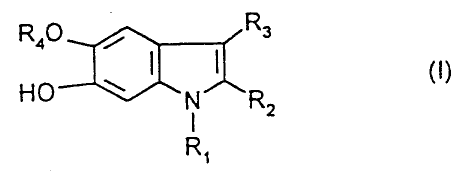 in which:
R₁, R₂, R₃, R₄, which are identical or different, designate a hydrogen atom or a C₁-C₄ alkyl radical, R₁, R₂, R₃ designating hydrogen when R₄ designates a C₁-C₄ alkyl radical, as well as the addition salts of these compounds with an acid.

10. Use according to Claim 9, characterized in that in the formula (I), the alkyl radical preferably designates methyl or ethyl and R₁, R₂ and R₃ do not simultaneously designate a C₁-C₄ alkyl radical.

11. Use according to either of Claims 9 and 10, characterized in that the compounds of formula (I) are chosen from 5,6-dihydroxyindole, 2-methyl-5,6-dihydroxyindole, 3-methyl-5,6-dihydroxyindole, 1-methyl-5,6-dihydroxyindole, 2,3-dimethyl-5,6-dihydroxyindole, 5-methoxy-6-hydroxyindole, and their addition salts with an acid.

12. Use according to Claim 11, characterized in that the compound of formula (I) is 5,6-dihydroxyindole.

13. Use according to any one of Claims 1 to 8, characterized in that the indoline compounds correspond to the following formula (II): 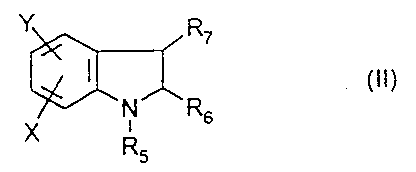 in which:
- R₅ and R₇, which are identical or different, represent a hydrogen atom or a C₁-C₄ alkyl radical;
- R₆ represents a hydrogen atom, a C₁-C₄ alkyl radical or -COOH;
- X designates a hydrogen atom, an amino, hydroxyl, C₁-C₄ alkyl or C₁-C₄ alkoxy radical;
- Y designates a hydroxyl or amino radical; with the proviso that when X designates a hydroxyl radical or an alkyl radical, X occupies the 5-, 6- or 7-positions and is in the ortho position relative to Y, as well as the addition salts of these compounds with an acid.

14. Use according to Claim 13, characterized in that the indoline compounds of formula (II) are chosen from 5,6-dihydroxyindoline, N-methyl-5,6-dihydroxyindoline, N-ethyl-5,6-dihydroxyindoline, N-butyl-5,6-dihydroxyindoline, 4-hydroxy-5-methoxyindoline, 6-hydroxy-7-methoxyindoline, 6,7-dihydroxyindoline, 4,5-dihydroxyindoline, 5-methoxy-6-hydroxyindoline, and their addition salts with an acid.

15. Use according to any one of Claims 1 to 14, characterized in that the indole and/or indoline compounds are used in proportions sufficient to repigment keratinous fibres.

16. Use according to Claim 15, characterized in that the indole and/or indoline compounds are used in proportions of between 0.1 and 5 % by weight relative to the total weight of the composition.

17. Use according to Claim 16, characterized in that the indole and/or indoline compounds are used in proportions of between 0.2 and 3 % by weight relative to the total weight of the composition.

18. Use according to any one of Claims 1 to 17, characterized in that the compositions are aqueous or consist of a mixture of water and of a cosmetically acceptable solvent.

19. Use according to Claim 18, characterized in that the solvent is chosen from C₁-C₄ lower alcohols, glycols, glycol ethers and lower esters.

20. Use according to any one of Claims 1 to 19, characterized in that the compositions have a pH of between 5 and 10.

21. Use according to Claim 20, characterized in that the compositions have a pH of between 7 and 9.5.

22. Use according to any one of Claims 1 to 21, characterized in that the compositions are provided in the form of lotions which are thickened to a greater or lesser degree, gels, shampoos, emulsions or foams.

23. Use according to any one of Claims 1 to 22, characterized in that the compositions contain at least one adjuvant chosen from anionic, cationic, non-ionic, amphoteric or zwitterionic surfactants or mixtures thereof, inorganic or organic thickening agents, antioxidants, penetrating agents, sequestrants, perfumes, buffers, dispersants, conditioners, film-forming agents, preservatives, opacifiers, sunscreens.

24. Use according to any one of Claims 1 to 23, characterized in that the compositions are applied to keratinous fibres and in particular to the hair with an exposure time generally of between 15 seconds and 30 minutes and then the hair is rinsed with water and dried.

## Patentansprüche

1. Verwendung eines Mangan-, Zink- und/oder Cobaltsalzes in einem einstufigen Verfahren zum Färben von Keratinfasern und insbesondere menschlichen Keratinfasern, wie dem Haar, unter Verwendung einer Indol- und/oder Inodlinverbindung, um Färbungen in grauen Farbschattierungen zu erhalten, die sich im Laufe der Zeit nicht verändern.

2. Verwendung nach Anspruch 1,
dadurch gekennzeichnet, daß
Färbungen in hellen grauen Farbschattierungen erhalten werden, die sich im Laufe der Zeit nicht verändern.

3. Verwendung nach Anspruch 1 oder 2,
dadurch gekennzeichnet, daß
das Mangan-, Zink- und/oder Cobaltsalz solubilisiert oder dispergiert in der Zusammensetzung, die die Indol- und/oder Indolinverbindung enthält, vorliegt.

4. Verwendung nach einem der Ansprüche 1 bis 3,
dadurch gekennzeichnet, daß
das Mangan-, Zink- und/oder Cobaltsalz, alleine oder im Gemisch verwendet wird und unter den Acetaten, Halogeniden, Sulfaten und Carbonaten von Mangan, Zink und/oder Cobalt sowie deren Hydraten ausgewählt ist.

5. Verwendung nach Anspruch 4,
dadurch gekennzeichnet, daß
das Mangan-, Zink- und/oder Cobaltsalz unter Mangandiacetattetrahydrat, Mangandichlorid und seinen Hydraten, Mangandihydrogencarbonat, Manganacetylacetonat, Mangantriacetat und seinen Hydraten, Mangantrichlorid, Zinkdichlorid, Zinkdiacetatdihydrat, Zinkcarbonat, Zinkdinitrat, Zinksulfat, Cobaltdiacetattetrahydrat, Cobaltcarbonat, Cobaltdichlorid, Cobaltdinitrat und Cobaltsulfatheptahydrat ausgewählt ist.

6. Verwendung nach Anspruch 5,
dadurch gekennzeichnet, daß
das Mangandiacetattetrahydrat oder das Zinkchlorid verwendet wird.

7. Verwendung nach einem der Ansprüche 1 bis 6,
dadurch gekennzeichnet, daß
die Mangan-, Zink- und/oder Cobaltsalze in Anteilen im Bereich von 0,01 bis 4 Gew.-%, ausgedrückt als Metall, bezogen auf das Gesamtgewicht der Zusammensetzung, verwendet werden.

8. Verwendung nach Anspruch 7,
dadurch gekennzeichnet, daß
die Mangan-, Zink- und/oder Cobaltsalze in Anteilen im Bereich von 0,005 bis 2 Gew.-%, ausgedrückt als Metall, bezogen auf das Gesamtgewicht der Zusammensetzung, verwendet werden.

9. Verwendung nach einem der Ansprüche 1 bis 8,
dadurch gekennzeichnet, daß
die Indolverbindungen der folgenden Formel (I) entsprechen: 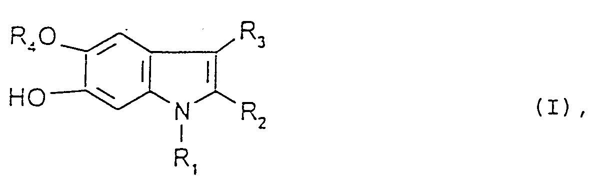 worin bedeuten:
R₁, R₂, R₃ und R₄, die gleich oder voneinander verschieden sind, Wasserstoff oder C₁₋₄-Alkyl, wobei R₁, R₂ und R₃ Wasserstoff bedeuten, wenn R₄ eine C₁₋₄-Alkylgruppe ist,
sowie die Additionssalze dieser Verbindungen mit einer Säure.

10. Verwendung nach Anspruch 9,
dadurch gekennzeichnet, daß
in Formel (I) die Alkylgruppe vorzugsweise Methyl oder Ethyl bedeutet, und R₁, R₂ und R₃ nicht gleichzeitig eine C₁₋₄-Alkylgruppe bedeuten.

11. Verwendung nach einem der Ansprüche 9 oder 10,
dadurch gekennzeichnet, daß
die Verbindungen der Formel (I) unter 5,6-Dihydroxyindol, 2-Methyl-5,6-dihydroxyindol, 3-Methyl-5,6-dihydroxyindol, 1-Methyl-5,6-dihydroxyindol, 2,3-Dimethyl-5,6-dihydroxyindol, 5-Methoxy-6-hydroxyindol und den Additionssalzen dieser Verbindungen mit einer Säure ausgewählt sind.

12. Verwendung nach Anspruch 11,
dadurch gekennzeichnet, daß
die Verbindung der Formel (I) das 5,6-Dihydroxyindol ist.

13. Verwendung nach einem der Ansprüche 1 bis 8,
dadurch gekennzeichnet,daß
die Indolinverbindungen der folgenden Formel (II) entsprechen: 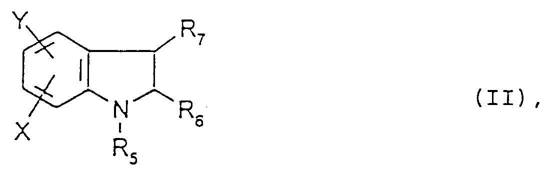 worin bedeuten:
- R₅ und R₇, die gleich oder voneinander verschieden sind, Wasserstoff oder C₁₋₄-Alkyl;
- R₆ Wasserstoff, C₁₋₄-Alkyl oder -COOH;
- X Wasserstoff, Amino, Hydroxy, C₁₋₄-Alkyl oder C₁₋₄-Alkoxy;
- Y Hydroxy oder Amino;
mit der Maßgabe, daß X in 5-, 6- oder 7-Position und in Bezug auf Y in o-Position substituiert ist, wenn X OH oder Alkyl bedeutet,
sowie die Additionssalze dieser Verbindungen mit einer Säure.

14. Verwendung nach Anspruch 13,
dadurch gekennzeichnet, daß
die Indolinverbindungen der Formel (II) unter 5,6-Dihydroxyindolin, N-Methyl-5,6-dihydroxyindolin, N-Ethyl-5,6-dihydroxyindolin, N-Butyl-5,6-dihydroxyindolin, 4-Hydroxy-5-methoxyindolin, 6-Hydroxy-7-methoxyindolin, 6,7-Dihydroxyindolin, 4,5-Dihydroxyindolin, 5-Methoxy-6-hydroxyindolin und den Additionssalzen dieser Verbindungen mit einer Säure ausgewählt sind.

15. Verwendung nach einem der Ansprüche 1 bis 14,
dadurch gekennzeichnet, daß
die Indol- und/oder Indolinverbindungen in Anteilen verwendet werden, die ausreichen, um die Keratinfasern zu repigmentieren.

16. Verwendung nach Anspruch 15,
dadurch gekennzeichnet, daß
die Indol- und/oder Indolinverbindungen in Anteilen im Bereich von 0,1 bis 5 Gew.-%, bezogen auf das Gesamtgewicht der Zusammensetzung verwendet werden.

17. Verwendung nach Anspruch 16,
dadurch gekennzeichnet, daß
die Indol- und/oder Indolinverbindungen in Anteilen im Bereich von 0,2 bis 3 Gew.-%, bezogen auf das Gesamtgewicht der Zusammensetzung, verwendet werden.

18. Verwendung nach einem der Ansprüche 1 bis 17,
dadurch gekennzeichnet, daß
die Zusammensetzungen wäßrige Zusammensetzungen sind oder aus einem Gemisch aus Wasser und einem kosmetisch akzeptablen Lösungsmittel bestehen.

19. Verwendung nach Anspruch 18,
dadurch gekennzeichnet, daß
das Lösungsmittel unter niederen C₁₋₄-Alkoholen, Glykolen, Glykolethern und niederen Estern ausgewählt ist.

20. Verwendung nach einem der Ansprüche 1 bis 19,
dadurch gekennzeichnet, daß
die Zusammensetzungen einen pH-Wert im Bereich von 5 bis 10 aufweisen.

21. Verwendung nach Anspruch 20,
dadurch gekennzeichnet, daß
die Zusammensetzungen einen pH-Wert im Bereich von 7 bis 9,5 aufweisen.

22. Verwendung nach einem der Ansprüche 1 bis 21,
dadurch gekennzeichnet, daß
die Zusammensetzungen in Form von mehr oder weniger dickflüssigen Lotionen, Gelen, Haarwaschmitteln, Emulsionen oder Schäumen vorliegen.

23. Verwendung nach einem der Ansprüche 1 bis 22,
dadurch gekennzeichnet, daß
die Zusammensetzungen mindestens einen Hilfsstoff enthalten, der unter anionischen, kationischen, nichtionischen, amphoteren, zwitterionischen grenzflächenaktiven Mitteln oder deren Gemischen, anorganischen oder organischen Verdickungsmitteln, Antioxidantien, Penetrationsmitteln, Maskierungsmitteln, Parfums, Puffern, Dispergiermitteln, Konditioniermitteln, Filmbildnern, Konservierungsmitteln, Trübungsmitteln und Sonnenschutzfiltern ausgewählt ist.

24. Verwendung nach einem der Ansprüche 21 bis 23,
dadurch gekennzeichnet, daß
die Zusammensetzung auf die Keratinfasern und insbesondere das Haar mit einer Einwirkungszeit im allgemeinen im Bereich von 15 s bis 30 min aufgetragen wird, und die Haare anschließend mit Wasser gespült und getrocknet werden.
